# EUROPÄISCHE PATENTANMELDUNG

(11) **EP 2 365 075 A1**
(43) Veröffentlichungstag der Anmeldung: **14.09.2011**
(21) Anmeldenummer: 10011812.4
(22) Anmeldetag: 09.01.2002
(51) Int. Cl.: C12N 15/11, A61K 31/713, C12N 15/88, A61P 35/00

(54) **Verfahren zur Hemmung der Expression eines Zielgens und Medikament zur Therapie einer Tumorerkrankung**

(30) Priorität: 09.01.2001 DE 10100586
(62) Teilanmeldung aus: 10002422.3
(71) Anmelder: Alnylam Europe AG, 95326 Kulmbach (DE)
(72) Erfinder: Kreutzer, Roland, Cambridge, MA 02142 (US); Limmer, Stefan, Cambridge, MA 02142 (US); Vornlocher, Hans-Peter, Cambridge, MA 02142 (US); Hadwigner, Philipp, Cambridge, MA 02142 (US); Geick, Anke, Cambridge, MA 02142 (US); Ocker, Matthias, Cambridge, MA 02142 (US); Herold, Christoph, Cambridge, MA 02142 (US); Schuppan, Detlef, Cambridge, MA 02142 (US)
(74) Vertreter: Vossius & Partner

(57) **Zusammenfassung**

Die Erfindung betrifft ein Verfahren zur Hemmung einer die Apoptose einer Tumorzelle hemmenden oder verhindernden Expression mindestens eines Zielgens, wobei mindestens eine doppelsträngige Ribonukleinsäure (dsRNA) in die Tumorzelle eingeführt wird, deren einer Strang S1 einen zum Zielgen zumindest abschnittsweise komplementären aus weniger als 25 aufeinanderfolgenden Nukleotiden bestehenden Bereich aufweist, wobei Verfahren zur therapeutischen Behandlung des menschlichen oder tierischen Körpers ausgeschlossen sind.

## Beschreibung

Die Erfindung betrifft ein Verfahren zur Hemmung der Expression mindestens eines Zielgens in einer Zelle sowie ein Medikament zur Therapie einer Tumorerkrankung.

Aus.der WO 99/32619 ist ein Verfahren zur Hemmung der Expression eines Zielgens mittels einem doppelsträngigen Oligoribonukleotid bekannt. Das bekannte Verfahren zielt auf die Hemmung der Expression von Genen in Zellen von Invertebraten ab. Dazu ist es erforderlich, daß das doppelsträngige Oligoribonukleotid eine zum Zielgen identische Sequenz mit einer Länge von mindestens 25 Basen aufweist.

Ein Verfahren zur Hemmung der Expression eines Zielgens in einer Zelle sowie ein Medikament sind aus der WO 00/44895 bekannt. Bei dem Verfahren wird ein Oligoribonukleotid mit doppelsträngiger Struktur (dsRNA) in die Zelle eingeführt. Ein Strang der dsRNA weist einen zum Zielgen zumindest abschnittsweise komplementären aus höchstens 49 aufeinanderfolgenden Nukleotidpaaren bestehenden Bereich auf. Das Medikament enthält mindestens eine dsRNA zur Hemmung der Expression eines vorgegebenen Zielgens, wobei ein Strang der dsRNA zum Zielgen zumindest abschnittsweise komplementär ist.

Aus Gautschi O. et al. (2001), J Natl Cancer Inst 93, Seiten 463 bis 471 ist es bekannt, daß eine erhöhte Expression der anti-apoptotisch wirkenden Proteine Bcl-2 und Bcl-xL an der Entwicklung und dem Fortschreiten vieler Tumore beteiligt ist. In Nacktmäusen erzeugte in vivo-Daten belegen, daß mit einer kombinierten Behandlung mit gegen die Expression von Bcl-2- und Bcl-xL-Genen gerichteten einzelsträngigen Antisinn-Oligonukleotiden ein Wachstum von Tumoren um etwa 50 bis 60% verringert werden konnte. Dazu war eine Behandlung mit 20 mg Oligonukleotiden pro Kilogramm Körpergewicht und Tag erforderlich. Durch diese große Menge erforderlicher Oligonukleotide ist die Behandlung verhältnismäßig teuer. Darüber hinaus können die eingesetzten einzelsträngigen Oligonukleotide schnell im Serum abgebaut werden. Die hohe Oligonukleotidmenge ist erforderlich, weil ein Antisinn-Oligonukleotid letztendlich in einer Menge in Zielzellen eingebracht werden muß, die mindestens genauso groß ist wie die Menge der dort vorhandenen mRNA des Zielgens. Mit dem Verfahren wurde lediglich eine Verringerung des Wachstums, nicht jedoch eine Rückbildung der Tumore erreicht.

Aufgabe der vorliegenden Erfindung ist es, die Nachteile nach dem Stand der Technik zu beseitigen. Es soll insbesondere ein Verfahren und ein Medikament angegeben werden, mit dem die Vermehrung von Tumorzellen effektiv und kostengünstig gehemmt werden kann.

Diese Aufgabe wird durch die Merkmale der Ansprüche 1 und 13 gelöst. Vorteilhafte Ausgestaltungen ergeben sich aus den Ansprüche 2 bis 12 und 14 bis 26.

Nach Maßgabe der Erfindung ist ein Verfahren zur Hemmung einer die Apoptose einer Tumorzelle hemmenden oder verhindernden Expression mindestens eines Zielgens vorgesehen, wobei mindestens eine doppelsträngige Ribonukleinsäure (dsRNA) in die Tumorzelle eingeführt wird, deren einer Strang S1 einen zum Zielgen zumindest abschnittsweise komplementären, aus weniger als 25 aufeinanderfolgenden Nukleotiden bestehenden Bereich aufweist. Unter dem "Zielgen" wird der DNA-Strang der doppelsträngigen DNA in der Tumorzelle verstanden, welcher komplementär zu einem bei der Transkription als Matrize dienenden DNA-Strang einschließlich aller transkribierten Bereiche ist. Bei dem Zielgen handelt es sich also im allgemeinen um den Sinn-Strang. Der Strang S1 kann somit komplementär zu einem bei der Expression des Zielgens gebildeten RNA-Transkript oder dessen Prozessierungsprodukt, wie z.B. einer mRNA, sein. Eine dsRNA liegt vor, wenn die aus einem oder zwei Ribonukleinsäure-Strängen bestehende Ribonukleinsäure eine doppelsträngige Struktur aufweist. Nicht alle Nukleotide der dsRNA müssen Watson-Crick-Basenpaarungen aufweisen. Insbesondere einzelne nicht komplementäre Basenpaare beeinträchtigen das Verfahren kaum oder gar nicht. Die maximal mögliche Zahl der Basenpaare ist die Zahl der Nukleotide in dem kürzesten in der dsRNA enthaltenen Strang. Unter "eingeführt werden" wird das Aufnehmen in die Zelle verstanden. Das Aufnehmen kann durch die Zelle selbst erfolgen. Es kann aber auch durch Hilfsstoffe oder Hilfsmittel vermittelt werden.

Es hat sich überraschenderweise gezeigt, daß mit diesem Verfahren die Vermehrung von Tumorzellen mit einer erheblich geringeren Menge an Oligoribonukleotiden gehemmt werden kann, als Oligonukleotide zum Erzielen eines vergleichbaren Ergebnisses bei der herkömmlichen Antisinn-Technik erforderlich sind. Darüber hinaus ist es mit dem erfindungsgemäßen Verfahren möglich, in einer Population von Tumorzellen in einem solchen Ausmaß Apoptosen auszulösen, daß nicht nur das Wachstum der Population, sondern die Gesamtzahl der Tumorzellen verringert wird. Bei Durchführung des Verfahrens mit normalen, d.h. nicht transformierten, Zellen bewirkt das Verfahren gegenüber einem mit einer Kontroll-dsRNA durchgeführten Verfahren keine signifikante Erhöhung der Apoptoserate. Die Kontroll-dsRNA ist eine dsRNA, welche keinen zu einem in den Zellen vorkommenden Gen komplementären Strang aufweist.

Als besonders vorteilhaft hat es sich erwiesen, wenn zumindest ein Ende der dsRNA einen aus 1 bis 4, insbesondere 2 oder 3, Nukleotiden gebildeten einzelsträngigen Überhang aufweist. Eine solche dsRNA weist gegenüber einer dsRNA ohne einzelsträngige Überhänge an mindestens einem Ende eine bessere Wirksamkeit bei der Hemmung der Expression des Zielgens auf. Ein Ende ist dabei ein Bereich der dsRNA, in welchem ein 5'- und ein 3'-Strangende vorliegen. Eine nur aus dem Strang S1 bestehende dsRNA weist demnach eine Schleifenstruktur und nur ein Ende auf. Eine aus dem Strang S1 und einem Strang S2 gebildete dsRNA weist zwei Enden auf. Ein Ende wird dabei jeweils von einem auf dem Strang S1 und einem auf dem Strang S2 liegenden Strangende gebildet.

Vorzugsweise befindet sich der einzelsträngige Überhang am 3'-Ende des Strangs S1. Diese Lokalisation des einzelsträngigen Überhangs führt zu einer weiteren Steigerung der Effizienz des Verfahrens. In einem Ausführungsbeispiel weist die dsRNA.nur an einem, insbesondere dem am 3'-Ende des Strangs S1 gelegenen, Ende einen einzelsträngigen Überhang auf. Das andere Ende ist bei einer zwei Enden aufweisenden dsRNA glatt, d.h. ohne Überhänge, ausgebindet. Eine solche dsRNA hat sich sowohl in verschiedenen Zellkulturmedien als auch in Blutserum als besonders beständig erwiesen.

Der komplementäre Bereich der dsRNA kann 19 bis 24, vorzugsweise 21 bis 23, insbesondere 22, Nukleotide aufweisen. Eine dsRNA mit dieser Struktur ist besonders effizient in der Inhibition des Zielgens. Der Strang S1 der dsRNA kann weniger als 30, vorzugsweise weniger als 25', besonders vorzugsweise 21 bis 24, Nukleotide aufweisen. Die Zahl dieser Nukleotide ist zugleich die Zahl der in der dsRNA maximal möglichen Basenpaare.

Mindestens ein Ende der dsRNA kann modifiziert werden, um einem Abbau in der Tumorzelle oder einer Dissoziation der doppelsträngigen Struktur entgegenzuwirken. Weiterhin kann der durch komplementäre Nukleotidpaare bewirkte Zusammenhalt der doppelsträngigen Struktur durch mindestens eine, vorzugsweise zwei, weitere chemische Verknüpfung/en erhöht werden. Die chemische Verknüpfung kann durch eine kovalente oder ionische Bindung, eine Wasserstoffbrückenbindung, hydrophobe Wechselwirkungen, vorzugsweise van-der-Waals- oder Stapelungswechselwirkungen, oder durch Metäll-Ionenkoordination gebildet werden. Sie kann auch durch in der doppelsträngigen Struktur anstelle.von Purinen benutzten Purinanaloga gebildet werden.

In einer bevorzugten Ausgestaltung des Verfahrens ist das Zielgen mindestens ein Gen der Bcl-2-Familie, insbesondere Bcl-2, Bcl-w oder Bcl-xL. Es ist auch möglich, daß mehrere Gene Zielgene sind. So können sowohl Bcl-2 als auch Bcl-xL Zielgene sein. Die Hemmung der Gene der Bcl-2-Familie ist besonders günstig, weil deren verstärkte Expression mit der Entwicklung und der Vermehrung vieler Tumorzellen in Zusammenhang steht. Die Hemmung mehrerer Zielgene ist vorteilhaft, weil es Tumorzellen gibt, welche mehrere anti-apoptotisch wirkende Gene exprimieren.

Vorzugsweise besteht die dsRNA aus einem Strang S2 mit der Sequenz SEQ ID NO: 1 und dem Strang S1 mit der Sequenz SEQ ID NO: 2 oder einem Strang S2 mit der Sequenz SEQ ID NO: 3 und dem Strang S1 mit der Sequenz SEQ ID NO: 4 gemäß dem anliegenden Sequenzprotokoll. Eine solche dsRNA ist in der Hemmung der Expression des Zielgens Bcl-2 besonders wirksam. Bei der Tumorzelle kann es sich um eine Pankreaskarzinomzelle handeln. Zum Einbringen der dsRNA in die Tumorzelle kann eine die dsRNA umschließende micellare Struktur, vorzugsweise ein Liposom, oder ein die dsRNA umschließendes Kapsid verwendet werden. Das Kapsid kann insbesondere ein virales natürliches Kapsid oder ein auf chemischem oder enzymatischem Weg hergestelltes künstliches Kapsid oder eine davon abgeleitete Struktur sein.

Weiterhin betrifft die Erfindung ein Medikament zur Therapie einer Tumorerkrankung, das mindestens eine doppelsträngige Ribonukleinsäure (dsRNA) zur Hemmung einer Expression mindestens eines Zielgens enthält, wobei ein Strang S1 der dsRNA einen zum Zielgen zumindest abschnittsweise komplementären aus weniger als 25 aufeinanderfolgenden Nukleotiden bestehenden Bereich aufweist. Das Zielgen ist dabei ein Gen, dessen Expression eine Apoptose von Tumorzellen hemmt oder verhindert. Das Medikament ist so zu dosieren, daß die Hemmung der Expression mindestens eines Zielgens erreicht werden kann. Überraschenderweise hat sich gezeigt, daß ein solches Medikament dazu sehr niedrig dosiert eingesetzt werden kann. Eine Dosierung von 5 mg dsRNA pro Kilogramm Körpergewicht und Tag sind ausreichend, um in den Tumorzellen eine Hemmung oder vollständige Unterdrückung der Expression des Zielgens zu erreichen. Bei einer solch niedrigen Dosierung werden Nebenwirkungen weitgehend ausgeschlossen.

Vorzugsweise weist zumindest ein Ende der dsRNA einen aus 1 bis 4, insbesondere 2. oder 3, Nukleotiden gebildeten einzelsträngigen Überhang auf. Der einzelsträngige Überhang kann sich am 3'-Ende des Strangs S1 befinden. Besonders bevorzugt weist die dsRNA nur an einem, insbesondere dem am 3'-Ende des Strangs S1 gelegenen, Ende einen einzelsträngigen Überhang auf. Es hat sich herausgestellt, daß eine solche dsRNA im Körper besonders beständig ist. Sie wird in Blut langsamer abgebaut bzw. ausgeschieden als eine dsRNA mit einzelsträngigen Überhängen an beiden Enden. Dadurch ist eine niedrige Dosierung möglich.

Der komplementäre Bereich kann 19 bis 24, bevorzugt 21 bis 23, insbesondere 22, Nukleotide aufweisen. Der Strang S1 kann weniger als 30, vorzugsweise weniger als 25, besonders vorzugsweise 21 bis 24, Nukleotide aufweisen. Bei einer Ausführungsform ist mindestens ein Ende der dsRNA modifiziert, um einem Abbau in den Tumorzellen oder einer Dissoziation entgegenzuwirken. Der durch komplementäre Nukleotidpaare bewirkte Zusammenhalt der doppelsträngigen Struktur kann durch mindestens eine, vorzugsweise zwei, weitere chemische Verknüpfung/en erhöht sein.

Das Zielgen ist vorzugsweise mindestens ein Gen der Bcl-2-Familie, insbesondere Bcl-2, Bcl-w oder Bcl-xL. Besonders effizient ist ein Medikament, welches eine sowohl für das Zielgen Bcl-2 als auch für das Zielgen Bcl-xL spezifische dsRNA aufweist.

Die dsRNA kann aus einem Strang S2 mit der Sequenz SEQ ID NO: 1 und dem Strang S1 mit der Sequenz SEQ ID NO: 2 oder einem Strang S2 mit der Sequenz SEQ ID NO: 3 und dem Strang S1 mit der Sequenz SEQ ID NO: 4 gemäß dem anliegenden Sequenzprotokoll bestehen. Die mit dem Medikament zu behandelnde Tumorerkrankung kann ein Pankreaskarzinom sein. Für das Pankreaskarzinom existiert bislang keine ausreichend erfolgreiche Therapie. Die 5-Jahres Überlebensrate liegt bei circa 3% und ist die niedrigste aller Karzinome. Die dsRNA kann in dem Medikament in einer Lösung oder von einer micellaren Struktur, vorzugsweise einem Liposom, oder einem Kapsid umschlossen vorliegen. Eine micellare Struktur oder ein Kapsid kann die Aufnahme der dsRNA in die Tumorzellen erleichtern. Das Medikament kann eine Zubereitung aufweisen, die zur Inhalation, oralen Aufnahme oder Injektion, insbesondere zur intravenösen oder intraperitonealen Injektion oder zur Injektion direkt in ein Tumorgewebe, geeignet ist. Eine zur Inhalation oder Injektion geeignete Zubereitung kann im einfachsten Fall aus einem physiologisch verträglichen Puffer, insbesondere einer phosphatgepufferten Salzlösung, und der dsRNA bestehen. Es hat sich nämlich überraschenderweise herausgestellt, daß eine lediglich in einem solchen Puffer gelöst dsRNA von den Tumorzellen aufgenommen wird und die Expression des Zielgens hemmt, ohne daß die dsRNA dazu in ein besonderes Vehikel verpackt werden muß.

Nachfolgend werden anhand der Figuren Beispiele der Erfindung erläutert. Es zeigen:
- Fig. 1: die prozentuale Apoptoserate von humanen Pankreaskarzinomzellen YAP C 120 Stunden nach Transfektion mit einer zu einer ersten Sequenz aus dem humanen Bcl-2-Gen komplementären dsRNA 1,
- Fig. 2: die prozentuale Apoptoserate der YAP C-Zellen 120 Stunden nach Transfektion mit einer zu einer zweiten Sequenz aus dem humanen Bcl-2-Gen komplementären dsRNA 2 und

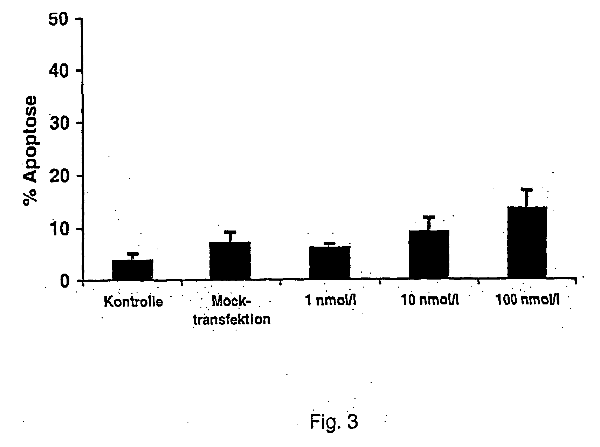

- Fig. 3: die prozentuale Apoptoserate von YAP C-Zellen 120 Stunden nach Transfektion mit einer zu einer Sequenz aus dem Noemycin-Resistenzgen komplementären dsRNA 3.

Zellen der humanen Pankreaskarzinomzellinie YAP C, welche unter der Nr. ACC 382 von der Deutschen Sammlung von Mikroorganismen und Zellkulturen, Braunschweig bezogen werden können, wurden unter konstanten Bedingungen bei 37°C, 5% CO₂ in RPMI 1640-Medium (Fa. Biochrom, Berlin) mit 10% fötalem Kälberserum (FKS) und 1% Penicillin/Streptomycin kultiviert. Humane Hautfibroblasten wurden unter den gleichen Bedingungen in Dulbecco's MEM mit 10% FKS und 1% Penicillin/Streptomycin kultiviert.

Die für Transfektionen eingesetzten doppelsträngigen Oligoribonukleotide weisen folgende, im Sequenzprotokoll mit SEQ ID NO:1 bis SEQ ID NO:6 bezeichneten, Sequenzen auf:
dsRNA 1, welche zu einer ersten Sequenz aus dem humanen Bcl-2-Gen komplementär ist:
   S2: 5'- cag gac cuc gcc gcu gca gac c-3' (SEQ ID NO: 1)
   S1: 3'-cg guc cug gag cgg cga cgu cug g-5' (SEQ ID NO: 2)
dsRNA 2, welche zu einer zweiten Sequenz aus dem humanen Bcl-2-Gen komplementär ist:
   S2: 5'- g ccu uug ugg aac ugu acg gcc-3' (SEQ ID NO: 3)
   S1: 3'-uac gga aac acc uug aca ugc cgg-5' (SEQ ID NO: 4)
dsRNA 3, welche zu einer Sequenz aus dem Neomycin-ResistenzGen komplementär ist:
   S2: 5'- c aag gau gag gau cgu uuc gca-3' (SEQ ID NO: 5)
   S1: 3'-ucu guc cua cuc cua gca aag cg -5' (SEQ ID NO: 6)

Die Transfektionen wurden in einer 6-Well-Platte mit Oligofectamine (Fa. Invitrogen, Karlsruhe) durchgeführt. Pro Well wurden 250.000 Zellen ausgesetzt. Die Transfektion der doppelsträngigen Oligoribonukleotide wurde nach dem von Invitrogen für Oligofectamine empfohlenen Protokoll durchgeführt (Angaben beziehen sich auf 1 Well einer 6-Well-Platte):
10 µl des doppelsträngigen Oligoribonukleotids (0,1 - 10 µM) wurden mit 175 µl Zellkulturmedium ohne Zusätze verdünnt. 3 µl Oligofectamine wurden mit 12 µl Zellkulturmedium ohne Zusätze verdünnt und 10 Minuten bei Raumtemperatur inkubiert. Das so verdünnte Oligofectamine wurde zu den bereits verdünnten doppelsträngigen Oligoribonukleotiden gegeben, gemischt und 20 Minuten bei Raumtemperatur inkubiert. In dieser Zeit wurden die zu transfizierenden Zellen einmal mit Zellkulturmedium ohne Zusätze gewaschen und 800 µl frisches Zellkulturmedium zugegeben. Danach wurden pro Well 200 µl des beschriebenen Oligofectarnine-dsRNA-Gemisches zugegeben, so daß das Endvolumen für die Transfektion 1000 µl betrug. Hierdurch ergibt sich eine Endkonzentration der doppelsträngigen Oligoribonukleotide von 1-100 µM. Der Transfektionsansatz wurde vier stunden bei 37°C bebrütet. Danach wurden pro Well 500 µl Zellkulturmedium mit 30% FKS zugegeben, so daß die Endkonzentration an FKS 10% betrug. Dieser Ansatz wurde für 120 Stunden bei 37°C inkubiert.

Nach der Inkubation wurden die Überstände gesammelt, die Zellen mit phosphatgepufferter Salzlösung (PBS) gewaschen, mittels Trypsin abgelöst und 10 Minuten mit 100 g zentrifugiert. Der Überstand wurde verworfen und das Pellet mit hypotoner Propidiumjodidlösung 30 Minuten bei 4°C im Dunkeln inkubiert. Die Analyse erfolgte durchflußzytometrisch in dem Fluoreszenz-unterstützten Zellsortierer FACSCalibur (Fa. BD GmbH, Heidelberg).

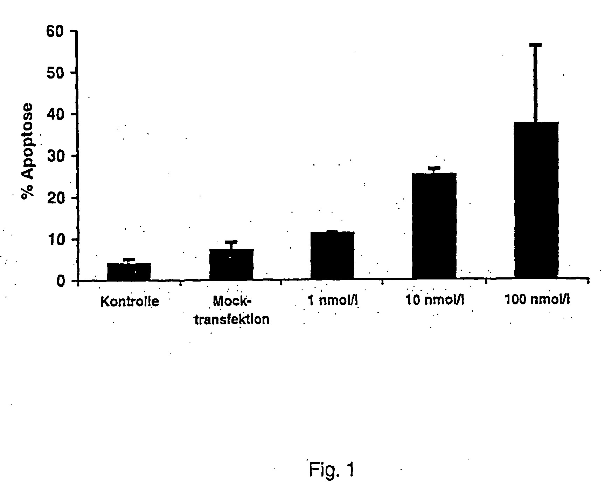

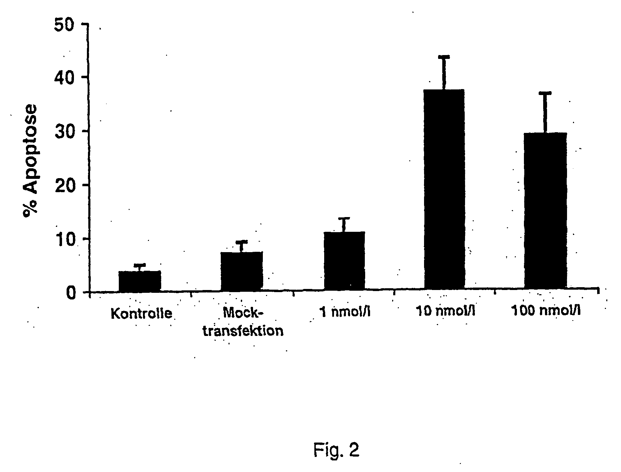

Die doppelsträngigen Oligoribonukleotide dsRNA 1 und dsRNA 2 verringern die durch Bcl-2 vermittelte Hemmung der Apoptose in den untersuchten humanen Pankreaskarzinomzellen. Zur Auslösung bzw. Einleitung der Apoptose ist keine zusätzliche Stimulation der Apoptose erforderlich. Die Apoptoserate stieg in Abhängigkeit von der Inkubationszeit an. Fig. 1 zeigt das mit der dsRNA 1 und Fig. 2 das mit der dsRNA 2 erzielte Ergebnis. Während unbehandelte YAP, C-Kontrollzellen und Zellen, mit welchen das beschriebene Verfahren zur Transfektion ohne doppelsträngiges Oligoribonukleotid durchgeführt wurde (mocktransfizierte Zellen), nur 3,8% und 7,1% Apoptose nach 120 Stunden Inkubation aufwiesen, konnte durch Transfektion mit 100 nM dsRNA die Apoptoserate nach 120 Stunden auf 37,2% bei Transfektion mit dsRNA 1 und 28,9% bei Transfektion mit dsRNA 2 gesteigert werden. Die Kontrolltransfektion mit der dsRNA 3 führte zu einer maximalen Apoptoserate von 13,5%. Das stellt keine signifikante Steigerung gegenüber den mocktransfizierten Zellen dar und belegt die Sequenzspezifität der Wirkung der dsRNAs 1 und 2.

Zur Kontrolle wurden auch Hautfibroblasten als nicht transformierte Zellen mit den dsRNAs 1 und 2 transfiziert. Diese Zellen zeigten nach 120 Stunden keinen signifikantes Ansteigen der Apoptoserate.

Die Anmeldung umfasst ferner die folgenden Ausführungsformen:
1. Verfahren zur Hemmung einer die Apoptose einer Tumorzelle hemmenden oder verhindernden Expression mindestens eines Zielgens, wobei mindestens eine doppelsträngige Ribonukleinsäure (dsRNA) in die Tumorzelle eingeführt wird, deren einer Strang S1 einen zum Zielgen zumindest abschnittsweise komplementären aus weniger als 25 aufeinanderfolgenden Nukleotiden bestehenden Bereich aufweist.
2. Verfahren nach Ausführungsform 1, wobei zumindest ein Ende der dsRNA einen aus 1 bis 4, insbesondere 2 oder 3, Nukleotiden gebildeten einzelsträngigen Überhang aufweist.
3. Verfahren nach einer der vorhergehenden Ausführungsformen, wobei sich der einzelsträngige Überhang am 3'-Ende des Strangs S1 befindet.
4. Verfahren nach einer der vorhergehenden Ausführungsformen, wobei die dsRNA nur an einem, insbesondere dem am 3'-Ende des Strangs S1 gelegenen, Ende einen einzelsträngigen Überhang aufweist.
5. Verfahren nach einer der vorhergehenden Ausführungsformen, wobei der komplementäre Bereich der dsRNA 19 bis 24, bevorzugt 21 bis 23, insbesondere 22, Nukleotide aufweist.
6. Verfahren nach einer der vorhergehenden Ausführungsformen, wobei der Strang S1 weniger als 30, vorzugsweise weniger als 25, besonders vorzugsweise 21 bis 24, Nukleotide aufweist.
7. Verfahren nach einer der vorhergehenden Ausführungsformen, wobei mindestens ein Ende der dsRNA modifiziert wird, um einem Abbau in der Tumorzelle oder einer Dissoziation entgegenzuwirken.
8. Verfahren nach einer der vorhergehenden Ausführungsformen, wobei der durch komplementäre Nukleotidpaare bewirkte Zusammenhalt der dsRNA durch mindestens eine, vorzugsweise zwei, weitere chemische Verknüpfung/en erhöht wird.
9. Verfahren nach einer der vorhergehenden Ausführungsformen, wobei das Zielgen mindestens ein Gen der Bcl-2 Familie, insbesondere Bcl-2, Bcl-w oder Bcl-xL, ist oder sowohl Bcl-2 als auch Bcl-xL Zielgene sind.
10. Verfahren nach einer der vorhergehenden Ausführungsformen, wobei die dsRNA aus einem Strang S2 mit der Sequenz SEQ ID NO: 1 und dem Strang S1 mit der Sequenz SEQ ID NO: 2 oder einem Strang S2 mit der Sequenz SEQ ID NO: 3 und dem Strang S1 mit der Sequenz SEQ ID NO: 4 gemäß dem anliegenden Sequenzprotokoll besteht.
11. Verfahren nach einer der vorhergehenden Ausführungsformen, wobei die Tumorzelle eine Pankreaskarzinomzelle ist.
12. Verfahren nach einer der vorhergehenden Ausführungsformen, wobei die dsRNA mittels einer die dsRNA umschließenden micellaren Struktur, vorzugsweise einem Liposom, oder eines die dsRNA umschließenden Kapsids, in die Tumorzelle eingebracht wird.
13. Medikament zur Therapie einer Tumorerkrankung enthaltend mindestens eine doppelsträngige Ribonukleinsäure (dsRNA) zur Hemmung einer die Apoptose von Tumorzellen hemmenden oder verhindernden Expression mindestens eines Zielgens, wobei ein Strang S1 der dsRNA einen zum Zielgen zumindest abschnittsweise komplementären aus weniger als 25 aufeinanderfolgenden Nukleotiden bestehenden Bereich aufweist.
14. Medikament nach Ausführungsform 13, wobei zumindest ein Ende der dsRNA einen aus 1 bis 4, insbesondere 2 oder 3, Nukleotiden gebildeten einzelsträngigen Überhang aufweist.
15. Medikament nach Ausführungsform 13 oder 14, wobei sich der einzelsträngige Überhang am 3'-Ende des Strangs S1 befindet.
16. Medikament nach einer der Ausführungsformen 13 bis 15, wobei die dsRNA nur an einem, insbesondere dem am 3'-Ende des Strangs S1 gelegenen, Ende einen einzelsträngigen Überhang aufweist.
17. Medikament nach einer der Ausführungsformen 13 bis 16, wobei der komplementäre Bereich 19 bis 24, bevorzugt 21 bis 23, insbesondere 22, Nukleotide aufweist.
18. Medikament nach einer der Ausführungsformen 13 bis 17, wobei der Strang S1 weniger als 30, vorzugsweise weniger als 25, besonders vorzugsweise 21 bis 24, Nukleotide aufweist.
19. Medikament nach einer der Ausführungsformen 13 bis 18, wobei mindestens ein Ende der dsRNA modifiziert ist, um einem Abbau in den Tumorzellen oder einer Dissoziation entgegenzuwirken.
20. Medikament nach einer der Ausführungsformen 13 bis 19, wobei der durch komplementäre Nukleotidpaare bewirkte Zusammenhalt der dsRNA durch mindestens eine, vorzugsweise zwei, weitere chemische Verknüpfung/en erhöht ist.
21. Medikament nach einer der Ausführungsformen 13 bis 20, wobei das Zielgen mindestens ein Gen der Bcl-2-Familie, insbesondere Bcl-2, Bcl-w oder Bcl-xL, ist oder sowohl Bcl-2 als auch Bcl-xL Zielgene sind.
22. Medikament nach einer der Ausführungsformen 13 bis 21, wobei die dsRNA aus einem Strang S2 mit der Sequenz SEQ ID NO: 1 und dem Strang S1 mit der Sequenz SEQ ID NO: 2 oder einem Strang S2 mit der Sequenz SEQ ID NO: 3 und dem Strang S1 mit der Sequenz SEQ ID NO: 4 gemäß dem anliegenden Sequenzprotokoll besteht.
23. Medikament nach einer der Ausführungsformen 13 bis 22, wobei die Tumorerkrankung ein Pankreaskarzinom ist.
24. Medikament nach einer der Ausführungsformen 13 bis 23, wobei die dsRNA in dem Medikament in einer Lösung oder von einer micellaren Struktur, vorzugsweise einem Liposom, oder einem Kapsid umschlossen vorliegt.
25. Medikament nach einer der Ausführungsformen 13 bis 24, wobei das Medikament eine Zubereitung aufweist, die zur Inhalation, oralen Aufnahme oder Injektion, insbesondere zur intravenösen oder intraperitonealen Injektion oder zur Injektion direkt in ein Tumorgewebe, geeignet ist.
26. Medikament nach Ausführungsform 25, wobei die Zubereitung aus einem physiologisch verträglichen Puffer, insbesondere einer phosphatgepufferten Salzlösung, und der dsRNA besteht.

## Patentansprüche

1. Verfahren zur Hemmung einer die Apoptose einer Tumorzelle hemmenden oder verhindernden Expression mindestens eines Zielgens, wobei mindestens eine doppelsträngige Ribonukleinsäure (dsRNA) in die Tumorzelle eingeführt wird, deren einer Strang S1 einen zum Zielgen zumindest abschnittsweise komplementären aus weniger als 25 aufeinanderfolgenden Nukleotiden bestehenden Bereich aufweist, wobei Verfahren zur therapeutischen Behandlung des menschlichen oder tierischen Körpers ausgeschlossen sind.

2. Verfahren nach Anspruch 1, wobei die Tumorzelle eine Pankreaskarzinomzelle ist.

3. Verfahren nach einem der vorhergehenden Ansprüche, wobei die dsRNA mittels einer die dsRNA umschließenden micellaren Struktur, vorzugsweise einem Liposom, oder eines die dsRNA umschließenden Kapsids, in die Tumorzelle eingebracht wird.

4. Medikament zur Verwendung in der Therapie einer Tumorerkrankung, enthaltend mindestens eine doppelsträngige Ribonukleinsäure (dsRNA) zur Hemmung einer die Apoptose von Tumorzellen hemmenden oder verhindernden Expression mindestens eines Zielgens, wobei ein Strang S1 der dsRNA einen zum Zielgen zumindest abschnittsweise komplementären aus weniger als 25 aufeinanderfolgenden Nukleotiden bestehenden Bereich aufweist.

5. Verfahren nach einem der Ansprüche 1 bis 3 oder Medikament zur Verwendung nach Anspruch 4, wobei beide Enden der dsRNA einen aus 1 bis 4, insbesondere 2 oder 3, Nukleotiden gebildeten einzelsträngigen Überhang aufweisen.

6. Verfahren nach einem der Ansprüche 1 bis 3 oder Medikament zur Verwendung nach Anspruch 4 oder 5, wobei sich ein einzelsträngiger Überhang am 3'-Ende des Strangs S1 befindet.

7. Verfahren nach einem der Ansprüche 1 bis 3 oder Medikament zur Verwendung nach einem der Ansprüche 4 bis 6, wobei der komplementäre Bereich 19 bis 24, bevorzugt 21 bis 23, insbesondere 22, Nukleotide aufweist.

8. Verfahren nach einem der Ansprüche 1 bis 3 oder Medikament zur Verwendung nach einem der Ansprüche 4 bis 7, wobei der Strang S1 weniger als 30, vorzugsweise weniger als 25, besonders vorzugsweise 21 bis 24, Nukleotide aufweist.

9. Verfahren nach einem der Ansprüche 1 bis 3 oder Medikament zur Verwendung nach einem der Ansprüche 4 bis 8, wobei mindestens ein Ende der dsRNA modifiziert ist, um einem Abbau in den Tumorzellen oder einer Dissoziation entgegenzuwirken.

10. Verfahren nach einem der Ansprüche 1 bis 3 oder Medikament zur Verwendung nach einem der Ansprüche 4 bis 9, wobei der durch komplementäre Nukleotidpaare bewirkte Zusammenhalt der dsRNA durch mindestens eine, vorzugsweise zwei, weitere chemische Verknüpfung/en erhöht ist.

11. Verfahren nach einem der Ansprüche 1 bis 3 oder Medikament zur Verwendung nach einem der Ansprüche 4 bis 10, wobei das Zielgen mindestens ein Gen der Bcl-2-Familie, insbesondere Bcl-2, Bcl-w oder Bcl-xL, ist oder sowohl Bcl-2 als auch Bcl-xL Zielgene sind.

12. Verfahren nach einem der Ansprüche 1 bis 3 oder Medikament zur Verwendung nach einem der Ansprüche 4 bis 11, wobei die dsRNA aus einem Strang S2 mit der Sequenz SEQ ID NO: 1 und dem Strang S1 mit der Sequenz SEQ ID NO: 2 oder einem Strang S2 mit der Sequenz SEQ ID NO: 3 und dem Strang S1 mit der Sequenz SEQ ID NO: 4 gemäß dem anliegenden Sequenzprotokoll besteht.

13. Medikament zur Verwendung nach einem der Ansprüche 4 bis 12, wobei die Tumorerkrankung ein Pankreaskarzinom ist.

14. Medikament zur Verwendung nach einem der Ansprüche 4 bis 13, wobei die dsRNA in dem Medikament in einer Lösung oder von einer micellaren Struktur, vorzugsweise einem Liposom, oder einem Kapsid umschlossen vorliegt.

15. Medikament zur Verwendung nach einem der Ansprüche 4 bis 14, wobei das Medikament eine Zubereitung aufweist, die zur Inhalation, oralen Aufnahme oder Injektion, insbesondere zur intravenösen oder intraperitonealen Injektion oder zur Injektion direkt in ein Tumorgewebe, geeignet ist.

16. Medikament zur Verwendung nach Anspruch 15, wobei die Zubereitung aus einem physiologisch verträglichen Puffer, insbesondere einer phosphatgepufferten Salzlösung, und der dsRNA besteht.


Sequenzprotokoll

<110>  Alnylam Europe AG

<120>  Verfahren zur Hemmung der Expression eines Zielgens und
       Medikament zu Therapie einer Tumorerkrankung

<130>  R2466 EP/2

<150>  DE 101 00 586.5
<151>  2001-01-09

<160>  6

<170>  PatentIn version 3.4

<210>  1
<211>  22
<212>  RNA
<213>  Künstliche Sequenz

<220>
<223>  Beschreibung der künstlichen Sequenz: Sinn-Strang einer zu einer
       Sequenz des humanen Bcl-2-Gens komplementären dsRNA

<400>  1
caggaccucg ccgcugcaga cc                                          22


<210>  2
<211>  24
<212>  RNA
<213>  Künstliche Sequenz

<220>
<223>  Beschreibung der künstlichen Sequenz: Antisinn-Strang einer zu
       einer Sequenz des humanen Bcl-2-Gens komplementären dsRNA

<400>  2
ggucugcagc ggcgaggucc uggc                                        24


<210>  3
<211>  22
<212>  RNA
<213>  Künstliche Sequenz

<220>
<223>  Beschreibung der künstlichen Sequenz: Sinn-Strang einer zu einer
       Sequenz des humanen Bcl-2-Gens komplementären dsRNA

<400>  3
gccuuugugg aacuguacgg cc                                          22


<210>  4
<211>  24
<212>  RNA
<213>  Künstliche Sequenz

<220>
<223>  Beschreibung der künstlichen Sequenz: Antisinn-Strang einer zu
       einer Sequenz des humanen Bcl-2-Gens komplementären dsRNA

<400>  4
ggccguacag uuccacaaag gcau                                        24

<210> 5
<211> 22
<212> RNA
<213> Künstliche Sequenz

<220>
<223> Beschreibung der künstlichen Sequenz: Sinn-Strang einer zu einer
Sequenz des Neomycin-Resistenzgens komplementären dsRNA

<400> 5
caaggaugag gaucguuucg ca 22


<210> 6
<211> 23
<212> RNA
<213> Künstliche Sequenz

<220>
<223> Beschreibung der künstlichen Sequenz: Antisinn-Strang einer zu
einer Sequenz des Neomycin-Resistenzgens komplementären dsRNA

<400> 6
gcgaaacgau ccucauccug ucu 23